# EUROPÄISCHE PATENTSCHRIFT

(11) **EP 1 563 833 B1**
(45) Veröffentlichungstag und Bekanntmachung des Hinweises auf die Patenterteilung: **02.11.2006**
(21) Anmeldenummer: 04106913.9
(22) Anmeldetag: 22.12.2004
(51) Int. Cl.: A61K 9/14, A61K 31/569

(54) **Pharmazeutische Zubereitungen mit amorphem Tibolon**
Pharmaceutical compositions with amorphous tibolone
Compositions pharmaceutiques comprenant du tibolone amorphe

(30) Priorität: 06.02.2004 EP 04002682
(43) Veröffentlichungstag der Anmeldung: 17.08.2005
(73) Patentinhaber: Helm AG, 20097 Hamburg (DE)
(72) Erfinder: Glänzer, Klaus, 22337, Hamburg (DE)
(74) Vertreter: Becker Kurig Straus

(56) Entgegenhaltungen:
- WO-A-03/000238
- WO-A-20/04045587
- WO-A-20/04103378

## Beschreibung

Die vorliegende Erfindung betrifft ein neuartiges Verfahren zur Herstellung von Wirkstoff-haltigen Pulvern, die amorphes Tibolon in morphologisch stabiler Form enthalten.. Die vorliegende Erfindung betrifft insbesondere ein Verfahren zur Herstellung von Tibolon-haltigen Wirkstoffpulvern, die dadurch charakterisiert sind, dass Tibolon hierin in amorpher Form vorliegt. Die Erfindung betrifft weiterhin pharmazeutische Formulierungen, zu deren Herstellung vorgenanntes Tibolon-haltiges Wirkstoffpulver eingesetzt werden kann. Bevorzugte erfindungsgemäße Arzneiformen sind Tabletten und Granulate, die auf an sich bekannte Weise mit üblichen, pharmazeutisch akzeptablen Hilfsstoffen hergestellt werden. Erfindungsgemäß besonders bevorzugt sind den Wirkstoff Tibolon schnell freisetzende Tabletten, die durch Direktverpressung des erfindungsgemäßen Wirkstoffpulvers, enthaltend Tibolon in amorpher Form, hergestellt werden.

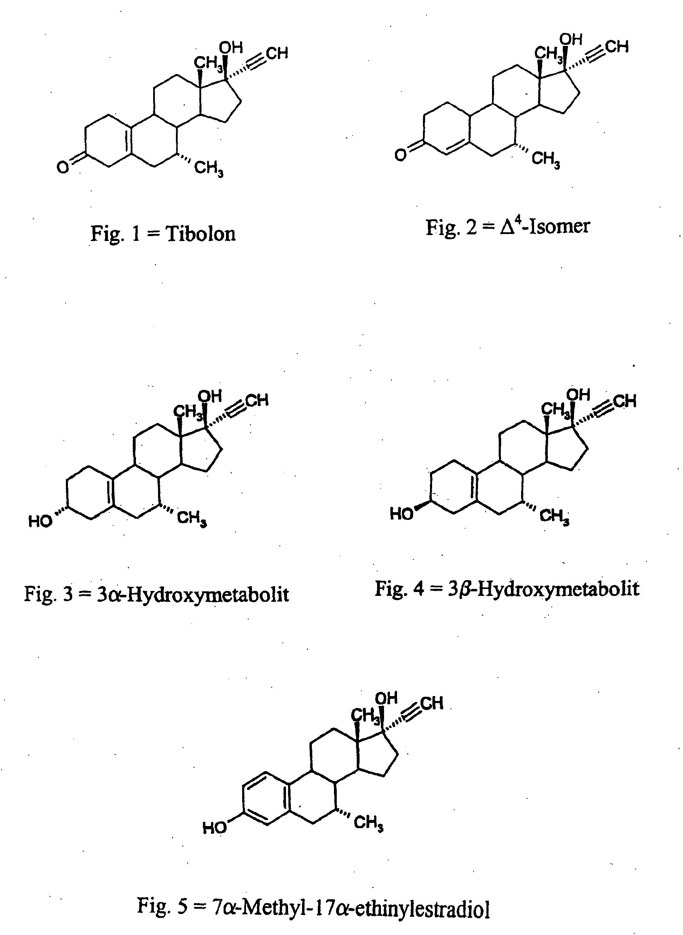

Der unter dem INN Tibolon bekannte Arzneistoff ist dem Chemiker unter dem Synonym 17-Hydroxy-7α-methyl-19-nor-17α-pregn-5(10)-en-20-in-3-on (IUPAC) (Formel siehe Figur 1) geläufig. Tibolon ist ein synthetisches Steroid, das dem Norethisteron ähnelt, welches eine kombinierte, estrogene, androgene und gestagene Wirkung besitzt. Die beiden Hydroxymetaboliten (3α- und 3β-) (Formeln siehe Figuren 3 und 4) haben eine estrogenähnliche Wirkung, während das Δ⁴-Isomer (Formel siehe Figur 2) durch Bindung an Gestagen- und Androgenrezeptoren überwiegend gestagene und androgene Wirkung zeigt (vgl. www.wechseljahre.com). Der vor kurzem identifizierte weitere Metabolit 7α-methyl-17α-ethinylestradiol (Formel siehe Figur 5) zeigt eine starke estrogene Wirkung (vgl. Steroids 67, 8 (2002), 681-686). Tibolon wird mit einer Tagesdosis von 2,5, unter anderem zur Hormonsubstitution in der Postmenopause, therapeutisch genutzt (siehe hierzu z.B. Drugs Fut. 6, 302 [1998]).

Tibolon ist eine instabile Substanz. Durch Einfluss von Wärme, saure oder oxidative Wechselwirkungen setzen entsprechende Abbauprozesse bereits während Synthese bzw. der Herstellung der Arzneiform ein. Letzteres ist besonders ausgeprägt bei allen Granulationsprozessen oder beim Versprühen von Tibolonlösungen. Auf diese Weise werden Abbau- und/oder Isomerisationsprozesse induziert, die eine unzureichende Stabilität des Endproduktes zur Folge haben. Um zu stabileren Formulierungen zu gelangen, wurden gemäß WO 03/032924 stabilisierende Zusätze mit pH-regulierenden und antioxidativen Eigenschaften vorgeschlagen und am Beispiel von Vor-Verreibungen mit Natriumbicarbonat bzw. Natriumcitrat und nachfolgender Feuchtgranulation beschrieben.

Eine weitere Besonderheit des Tibolons stellt neben der bereits erwähnten Umwandlung in stereoisomere Verbindungen im gelösten Zustand auch dessen Morphologie beim Ausfällen oder Auskristallisieren dar. Je nach Trocknungs-, Ausfällungs- oder Kristallisationsbedingungen fallen unterschiedliche Feststoffe/Morphologien an. Abhängig von den Lösungsmittelsystemen kann man z.B. aus ausgewählten polaren Lösungsmitteln vorwiegend Polymorph I, aus ausgewählten unpolaren Lösungsmitteln vorwiegend Polymorph II, oder ansonsten Mischungen davon erhalten (EP 0389035A1). Kristallisationsprozess und Phasenübergänge sind von einer Vielzahl schwer kontrollierbarer Faktoren abhängig (z.B. S. X. M. Boerrigter et al., J. Phys. Chem. B., 4725-4731 [2002]; 14th Int. Symp. on Industrial Crystallization, Cambridge [1999]; EP-A-389 035). Insbesondere ist festzustellen, dass geringfiigige Änderungen der Kristallisationsbedingungen, der Lösungsmittelsysteme, des Sättigungsgrades, von Impfkristallen und Trocknungsbedingungen, die Ausbildung einer bestimmten Morphologie stark beeinflussen.

Ausserdem ist festzustellen, dass Tibolon in amorpher, morphologisch stabiler Form bis jetzt weder als Substanz noch als Inhaltsstoff pharmazeutischer Formulierungen beschrieben wurde. Dies ist nach Erkenntnissen des Erfmders der vorliegenden Anmeldung auf die für diese Substanz typische sehr schnelle Umwandlung in kristalline Formen zurückzuführen.

Zu diesen unsicheren und teilweise nicht genau definierbaren morphologischen Verhältnissen des Wirkstoffes stellt die bei bestimmten Verhältnissen mögliche Phasenumwandlung im Verlauf längerer Lagerung der Formulierungen ein weiteres schwer kontrollierbares Qualitätsproblem dar.

In diesem Zusammenhang ist zu bedenken, dass unterschiedliche Polymorphe unterschiedliches Lösungsverhalten aufweisen. So weisen z.B. die Tibolon-Kristalle der Form II eine gegenüber Form I geringere Auflöserate auf. Dies wiederum führt zu unterschiedlichen, nicht gleichbleibenden Dissolutionsprofilen der daraus hergestellten Arzneien (siehe EP-A-389 035). Zusammenfassend kann daher festgestellt werden, dass ganz besonders bei Verfahren zur Herstellung pharmazeutischer Zubereitungen, bei denen Tibolon-Wirkstoff vor- oder angelöst werden muss, ein sehr hohes Risiko der unkontrollierten Umwandlung in polymorphe oder isomere Formen besteht, so dass die Erfüllung regulatorischer Anforderungen an Arzneimittel sowohl hinsichtlich eines klar definierten wirksamen Bestandteils als auch hinsichtlich der gleichbleibenden Qualität des Arzneimittels nicht gewährleistet ist.

Daher ist es Aufgabe der vorliegenden Erfindung, ein einfaches und kostengünstiges Verfahren zur Herstellung oraler Tibolon-Formulierungen zu entwickeln, die die oben beschriebenen Nachteile vermeiden.

Weiterhin ist es Aufgabe dieser Erfindung, Wirkstoff-haltige Pulver, die amorphes Tibolon in morphologisch stabiler Form enthalten, zu entwickeln.

Weiterhin ist es Aufgabe dieser Erfindung, ein Herstellungsverfahren zur Gewinnung dieser Wirkstoff-haltigen Pulver, die amorphes Tibolon in morphologisch stabiler Form enthalten, zu entwickeln.

Weiterhin ist es Aufgabe dieser Erfindung, ein Verfahren zur Herstellung oraler pharmazeutischer Formulierungen in Form von Tabletten und Granulaten, die auf den oben genannten Tibolon-haltigen Pulvern basieren, zu entwickeln.

Die Aufgabenstellung wird erfindungsgemäß durch ein Verfahren zur Herstellung von Pulvern, enthaltend amorphes Tibolon, gemäss Anspruch 1 gelöst.. Dabei geht man von einer Lösung des Tibolons in einem vorwiegend organischen Lösungsmittel aus, suspendiert und/oder löst darin das Trägermaterial und entfernt anschliessend das Lösungsmittel auf geeignete Weise, insbesondere durch Trocknen. Der Gesamtwassergehalt des Lösungsmittels beträgt erfindungsgemäß nicht mehr als 15 Vol.-%, bevorzugt nicht mehr als 5 Vol.-% (vergleiche Anspruch 1).

Erfindungsgemäße Trägermaterialien sind pharmazeutisch akzeptable Acrylsäure-Polymere und Acrylsäure-Copolymere. Bevorzugte Trägermaterialien sind Polymethacrylate, die als Eudragite^{®} kommerziell erhältlich sind (siehe hierzu Fiedler, Lexikon der Hilfsstoffe, Verlag Editio Cantor [2000]. S. 698 - S. 690), besonders bevorzugte Trägermaterialien sind Amminoalkylmethacrylate.

Erfindungsgemäß wird ein Verhältnis von Wirkstoff, d. h. von Tibolon zu Träger im Bereich von 1:0,1 bis 10, insbesondere im Bereich von 1:0,5 bis 1:5 eingestellt.

Die Erfindung betrifft weiterhin pharmazeutische Formulierungen, enthaltend diese neuen Wirkstoff-haltigen Pulver mit amorphem Tibolon in morphologisch stabiler Form. Die erfindungsgemäßen pharmazeutischen Formulierungen enthalten gegebenenfalls weitere Hilfsstoffe und können in die gewünschte Darreichungsform überführt werden. Besonders bevorzugt sind den Wirkstoff schnell freisetzende, durch Direktverpressung hergestellte Tabletten, die gegebenenfalls beschichtet werden können.

Für das erfindungsgemäße Verfahren zur Herstellung der Wirkstoff-haltigen Pulver, die amorphes Tibolon in morphologisch stabiler Form enthalten, eignen sich organische Lösungsmittel für die den pharmazeutischen Wirkstoff enthaltende Lösung. Die organischen Lösungsmittel sind insbesondere ausgewählt aus der Gruppe der niederen Alkanole mit 1 bis 4 Kohlenstoffatomen, der Gruppe der Ether, der Ester, der Gruppe der mehrwertigen Alkohole wie z.B. Glykol, und der Gruppe der aliphatischen Ketone und Gemische der vorgenannten Lösungsmittel. Besonders bevorzugt sind Methanol, Ethanol, Isopropanol, n-Propanol, Aceton und andere Lösungsmittel wie Ethylacetat, Methylethylketon, MTBE (Methyl-t.-butylester), Dichlormethan, Petrolether, Hexan, ein Aceton/Wasser-Gemisch, ein Aceton/Hexan-Gemisch, ein Ethylacetat/Hexan-Gemisch, Dichlormethan/Ethylacetat, sowie Gemische der vorgenannten Lösungsmittel. Erfindungsgemäß wird bevorzugt unter Inertgasatmosphäre, wie z. B. unter Stickstoffatmosphäre gearbeitet und, falls dies notwendig ist, werden entgaste bzw. sauerstofffreie Lösungen verwendet. Die Lösungen können auch Zusätze basischer Stoffe wie flüchtige Stickstoffbasen (z.B. Alkylamine oder auch Pyridin), oder Antioxidantien (z. B. Ascorbylpalmitat, Buthylhydroxytoluol, Buthylhydroxyanisol) enthalten.

Für die Herstellung der pharmazeutischen Formulierungen, wobei insbesondere Tabletten bevorzugt sind, können alle üblichen pharmazeutischen Hilfsstoffe verwendet werden. Als Füllstoffe und/oder Bindemittel können, z.B. Cellulosen und/oder Cellulosederivate (z. B. mikrokristalline Cellulose, native Cellulose, Hydroxypropylcellulose, Hydroxypropylmethylcellulose, Methylcellulose), Zucker (z.B. Lactose, Fructose, Saccharose, Glucose, Maltose), Zuckeralkohole (z.B. Lactit, Mannit, Sorbit, Xylit), anorganische Füllstoffe (z.B. Calciumphosphate und Calciumsulfate) und Stärken (z.B. Maisstärke, Kartoffelstärke, Weizenstärke, Dextrine, pregelatinisierte Stärken) verwendet werden. Darüber hinaus können alle weiteren Hilfsstoffe, die dem Fachmann aufgrund seines galenischen Basiswissens bekannt sind, wie z.B. Schmiermittel, Zerfallhilfsmittel, Netzmittel, Stoffe zur Verbesserung des Fliessverhaltens, alkalische Zusatzstoffe, Stabilisatoren sowie Aromen, Farbpigmente und Farbstoffe zur Herstellung der erfindungsgemäßen Arzneiformulierungen eingesetzt werden.

Der Anteil an Bindemitteln in der Gesamtmischung der Arzneimittelzubereitung ist bevorzugt 0 bis 20 % (m/m), der Anteil an Füll- und Hilfsstoffen in der Gesamtmischung beträgt 20 bis 99 Gew.-%, bevorzugt von 50 bis 99 Gew.-%.

Mit dem erfindungsgemäßen Verfahren werden insbesondere auch bezüglich ihrer Morphologie beständige Tibolon-Zubereitungen hergestellt, die zudem durch eine schnelle Wirkstofffreisetzung in Wasser unter physiologischen Bedingungen charakterisiert sind. Es wurde erfindungsgemäß ein Verfahren gefunden, das ausgehend von einer Lösung des Tibolons in einem organischen Lösungsmittel zu direkt weiterverarbeitbarem Pulver mit amorphem Wirkstoff führt.

Die Tibolon-Wirkstofflösung kann grundsätzlich in einer Ausführungsform der Erfindung durch Auflösen des Tibolons in einem geeigneten organischen Lösungsmittel hergestellt werden; vorteilhafter ist jedoch, eine im Rahmen der Synthese ohnehin anfallende Wirkstofflösung ohne Isolierung des Tibolons direkt zu verwenden.

Beispielsweise kann das Tibolon gemäß Wieland und Anner, Helv. Chim. Acta, 1453-1461 [1967] hergestellt werden, wobei dann auf Rekristallisierungsschritte durch Lösen in Dichlormethan/Ether verzichtet wird, und statt dessen das Trägermaterial in der Wirkstofflösung gelöst und das Lösungsmittel später entfernt wird. Die Art des organischen Lösungsmittels ergibt sich dann im Einzelfall aus dem abschließenden Synthese-Schritt der Wirkstoflherstellung.

Der Trocknungsvorgang kann durch Temperieren und Vakuum unterstützt werden. Gleichermaßen kann der Trocknungsvorgang als Gefriertrocknung erfolgen (siehe nachfolgende Beispiele). Das Lösungsmittel kann durch Arbeiten im geschlossenen System zurückgewonnen und für den Folgeprozess wieder eingesetzt werden. Erfindungsgemäß ist, dass eine Ausfällung und Isolierung des Tibolons entfällt. Gemäß dem beschriebenen erfindungsgemäßen Verfahren hergestellte Tibolon-haltige Zubereitungen können direkt zur Weiterverarbeitung zu Arzneiformen wie Tabletten oder Granulaten eingesetzt werden, bevorzugt zur Weiterverarbeitung mittels eines Direktkompressionsverfahrens.

Gegebenenfalls können die so erhaltenen Arzneiformen für spezielle Anwendungen mit den üblichen Filmüberzügen, z.B. zur Freisetzungssteuerung und/oder zur Geschmacksmaskierung und/oder zur Verbesserung der Stabilität versehen werden, wie dies z.B. in Kapitel 9 der Monographie von W.A. Ritschel, Die Tablette, Verlag Editio Cantor [2002] und in den dort zitierten Literaturstellen beschrieben wird.

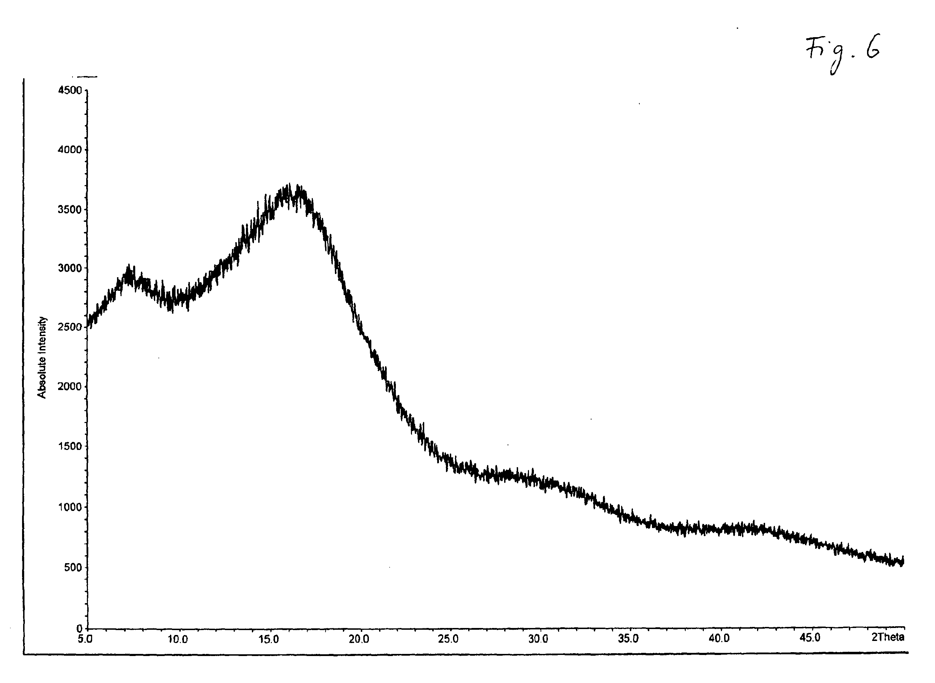

Überraschenderweise wurde gefunden, dass nach dem erfindungsgemäßen Verfahren hergestellte Zubereitungen den Wirkstoff in der für Arzneimittel erforderlichen homogenen Verteilung aufweisen und diesen uneingeschränkt freisetzen. Die bisher bekannten Kristallstrukturen der beiden Modifikationen des Tibolons (monoklin und triklin), oder gegebenenfalls andere kristalline Formen, sind bei den erfindungsgemäßen Zubereitungen nicht auffindbar. Vielmehr werden stets rein amorphe Strukturen des Tibolons erhalten (siehe Figuren 6 bis 9).

Die genannten Eigenschaften bleiben auch insbesondere dann erhalten, wenn die Tibolon-Zubereitungen zu Arzneiformen, wie z.B. Tabletten verarbeitet werden. Darüber hinaus führt diese direkte Verarbeitung zu keiner Veränderung des Gehalts an Neben- und Abbauprodukten (=Summe aller Verunreinigungen) auf dem Wege vom Wirkstoff zur Arzneiform (Tablette).

Die Erfindung wird nun anhand der folgenden Beispiele und Figuren näher erläutert.

### Beispiele 1 bis 4

### Verwendete Analysemethoden

### 1. HPLC-Methode zur Bestimmung des Wirkstoffgehaltes bzw. der Summe aller Verunreinigungen

Alle HPLC-Messungen wurden mit einem Agilent 100-HPLC durchgeführt.

| | |
|---|---|
| Verwendete Säule: | CC 250/4 Nucleosil 100-5 C18 |
| Mobile Phase: | 50 % wässerige Ammoniumbicarbonat-Lösung (0,005 %) |
| | 50 % Acetonitril |
| Fliessgeschwindigkeit: | 1,0 ml min⁻¹ |
| Detektor: | UV, 210 nm |
| Injektionsvolumen: | 20 µl |
| Retentionszeit Tibolon: | ca. 13 Min. |
| Analysedauer: | 60 Min. |

### 2. Wirkstoff-Freisetzung (Dissolutionstest) gemäß Ph. Eur., 1000 ml Wasser, 0,25 % Natriumlaurylsulfat (Soll: mind. 80 % Freisetzung nach 30 Min.)

### 3. Die Pulverröntgenbeugungsdiagramme wurden wie folgt gemessen:

| | |
|---|---|
| Gerät: | STADI P Transmissions-Diffraktometer |
| | Cu-Ka₁-Strahlung (1 = 1.54056 Ǻ), U = 40 kV, I = 30 mA Sekundärstrahl-Monochromator (eben, Graphit) Detektor: Scintillactionszähler |
| Spaltbreite: | 2 x 8 mm; 0,7 mm; 0,35 mm |
| Linear PSD: | 2θ = 2° bis 35°, 5 s / 0,04° stufenweise |
| Probe: | Pulver, Reflektionsmodus |

### Figuren 1 bis 9

Es zeigen
- Figur 1: die Tibolon-Strukturformel,
- Figur 2: die Strukturformel des Δ⁴-Isomers von Tibolon,
- Figur 3: die Strukturformel des 3α-Hydroxymetabolits von Tibolon,
- Figur 4: die Strukturformel des 3β-Hydroxymetabolits von Tibolon,
- Figur 5: die Strukturformel von 7α-Methyl-17α-ethinylestradiol, einem Tibolonmetaboliten,
- Figur 6: ein Pulverröntgenbeugungsdiagramm eines erfindungsgemäßen amorphen Tibolon-Pulvers, welches gemäß Beispiel 1 hergestellt wurde,
- Figur 7: ein Pulverröntgenbeugungsdiagramm eines erfindungsgemäßen amorphen Tibolon-Pulvers, welches gemäß Beispiel 3 hergestellt wurde,
- Figur 8: ein Pulverröntgenbeugungsdiagramm eines erfindungsgemäßen amorphen Tibolon-Pulvers, welches gemäß Beispiel 4 hergestellt wurde,
- Figur 9: ein Pulverröntgenbeugungsdiagramm eines erfindungsgemäßen amorphen Tibolon-Pulvers, welches gemäß Beispiel 4 hergestellt wurde, nach 3 Monaten Lagerung bei 30° C.

### Beispiel 1: Herstellung von Tabletten mit amorphem Tibolon, Rezeptur 1 (erfindungsgemäß)

Zu einer Lösung von 1 g kristallinem Tibolon in 16 ml Aceton werden 2 g Polymethacrylat (Eudragit^{®} E) gegeben und unter leichtem Erwärmen (30 bis 40° C) gelöst. Die klare Lösung wird gefriergetrocknet, so dass ein feines Wirkstoff-haltiges Pulver, enthaltend amorphes Tibolon (nachfolgend "Tibolon-Pulver" genannt) erhalten wird. Das Röntgenbeugungsdiagramm des Tibolon-Pulvers ist in Abbildung 6 dargestellt; es weist nur amorphe Anteile auf. Das Tibolon-Pulver wird mit pharmazeutischen Hilfsstoffen gemäß folgender Rezeptur vermischt und zu Tabletten verpresst:

| | |
|---|---|
| ■ Tibolon-Pulver, entsprechend 2,5 mg Tibolon | 7,5 mg |
| ■ Mikrokristalline Cellulose (Celphere SCP-100^{®}) | 77,5 mg |
| ■ Hilfsstoffe (Croscarmellose-Natrium, Natriumlaurylsulfat, Siliziumdioxid, Magnesiumstearat) in den üblichen Mengen | 15 mg |

Die verwendeten Mengen der weiteren Hilfsstoffe sind dem Fachmann aufgrund seines Basiswissens bekannt und können Standardwerken zur Formulierung von Tabletten, wie z.B. Ritschel et al., die Tablette, Editio Cantor - Aulendorf, 2. Auflage [2002] entnommen werden.

**Eigenschaften der pressfertigen Mischung und der Tabletten:**

| | |
|---|---|
| ■ Kompressibilität und Fließfähigkeit: | gut |
| ■ Mittlere Härte: | 91 N |
| ■ Abrieb: | < 0,1 % (bestimmt nach Ph. Eur.) |
| ■ Zerfallzeit: | 68 Sek. (bestimmt nach Ph. Eur.) |
| ■ Freisetzung: | 88 % nach 15 Min. |
| ■ Wirkstoffgehalt: | 2,53 % Tibolon |
| ■ Gleichförmigkeit des Gehaltes: | entspricht Ph. Eur. |
| ■ Summe aller Verunreinigungen (bezogen auf Tibolon-Peak): | 0,11 % |

Die so erhaltenen Tabletten können gegebenenfalls mit einem Überzug versehen werden.

### Beispiel 2: Herstellung von Tabletten mit amorphem Tibolon, Rezeptur 2 (erfindungsgemäß)

Zu einer Lösung von 1 g kristallinem Tibolon in 18 ml Aceton/Ethanol (3:1) werden 2 g Polymethacrylat (Eudragit^{®} E) gegeben und unter leichtem Erwärmen (30 bis 40° C) gelöst. Die klare Lösung wird gefriergetrocknet, so dass ein feines Wirkstoff-haltiges Pulver, enthaltend amorphes Tibolon (nachfolgend "Tibolon-Pulver" genannte) erhalten wird. Das daraus erhaltene Tibolon-Pulver wird mit pharmazeutischen Hilfsstoffen gemäß folgender Rezeptur vermischt und zu Tabletten verpresst:

| | |
|---|---|
| ■ Tibolon-Pulver, entsprechend 2,5 mg Tibolon | 7,5 mg |
| ■ Mannit (direkt verpressbar) | 77,5 mg |
| ■ Hilfsstoffe (wie in Beispiel 1) | 15 mg |

**Eigenschaften der pressfertigen Mischung und der Tabletten:**

| | |
|---|---|
| ■ Kompressibilität und Fließfähigkeit: | befriedigend bis gut |
| ■ Mittlere Härte: | 101 N |
| ■ Abrieb: | 0,1 % (bestimmt nach Ph. Eur.) |
| ■ Zerfallzeit: | 80 Sek. (bestimmt nach Ph. Eur.) |
| ■ Freisetzung: | 92 % nach 15 Min. |
| ■ Wirkstoffgehalt: | 2,47 % Tibolon |
| ■ Gleichförmigkeit des Gehaltes: | entspricht Ph. Eur. |
| ■ Summe aller Verunreinigungen (bezogen auf Tibolon-Peak): | 0,12 % |

Die so erhaltenen Tabletten können gegebenenfalls mit einem Überzug versehen werden.

### Beispiel 3: Herstellung von Tabletten mit amorphem Tibolon, Rezeptur 3 (erfindungsgemäß)

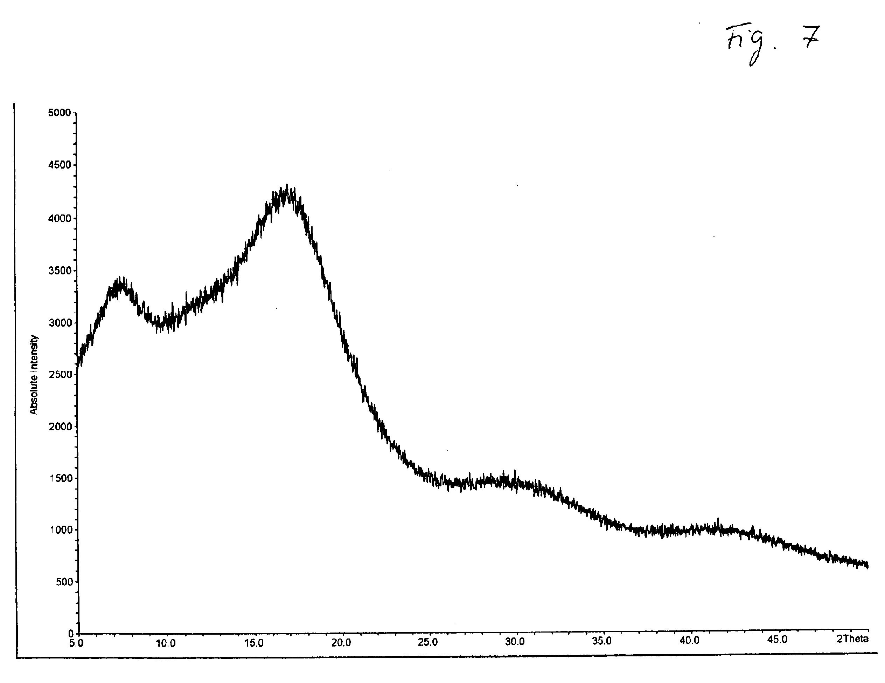

Zu einer Lösung von 1 g kristallinem Tibolon in 20 ml Aceton werden 4 g Polymethacrylat (Eudragit^{®} E) gegeben und unter leichtem Erwärmen (30 bis 40° C) gelöst. Die klare Lösung wird gefriergetrocknet, so dass ein feines Wirkstoff-haltiges Pulver, enthaltend amorphes Tibolon (nachfolgend "Tibolon-Pulver" genannt) erhalten wird. Das Röntgenbeugungsdiagramm des daraus erhaltenen feinen Pulvers ist in Abbildung 7 dargestellt; es weist nur amorphe Teile auf. Das Tibolon-Pulver wird mit pharmazeutischen Hilfsstoffen gemäß folgender Rezeptur vermischt und zu Tabletten verpresst:

| | |
|---|---|
| ■ Tibolon-Pulver, entsprechend 2,5 mg Tibolon | 12,5 mg |
| ■ Lactose (direkt verpressbar) | 72,5 mg |
| ■ Hilfsstoffe (wie in Beispiel 1) | 15 mg |

**Eigenschaften der pressfertigen Mischung und der Tabletten:**

| | |
|---|---|
| ■ Kompressibilität und Fließfähigkeit: | befriedigend bis gut |
| ■ Mittlere Härte: | 81 N |
| ■ Abrieb: | 0,1 % (bestimmt nach Ph. Eur.) |
| ■ Zerfallzeit: | 91 Sek. (bestimmt nach Ph. Eur.) |
| ■ Freisetzung: | 84 % nach 15 Min. |
| ■ Wirkstoffgehalt: | 2,52 % Tibolon |
| ■ Gleichförmigkeit des Gehaltes: | entspricht Ph. Eur. |
| ■ Summe aller Verunreinigungen (bezogen auf Tibolon-Peak): | 0,11 % |

Die so erhaltenen Tabletten können ebenfalls mit einem Überzug versehen werden.

### Beispiel 4: Stabilität von erfindungsgemäßem amorphen Tibolon-Pulver (erfindungsgemäß)

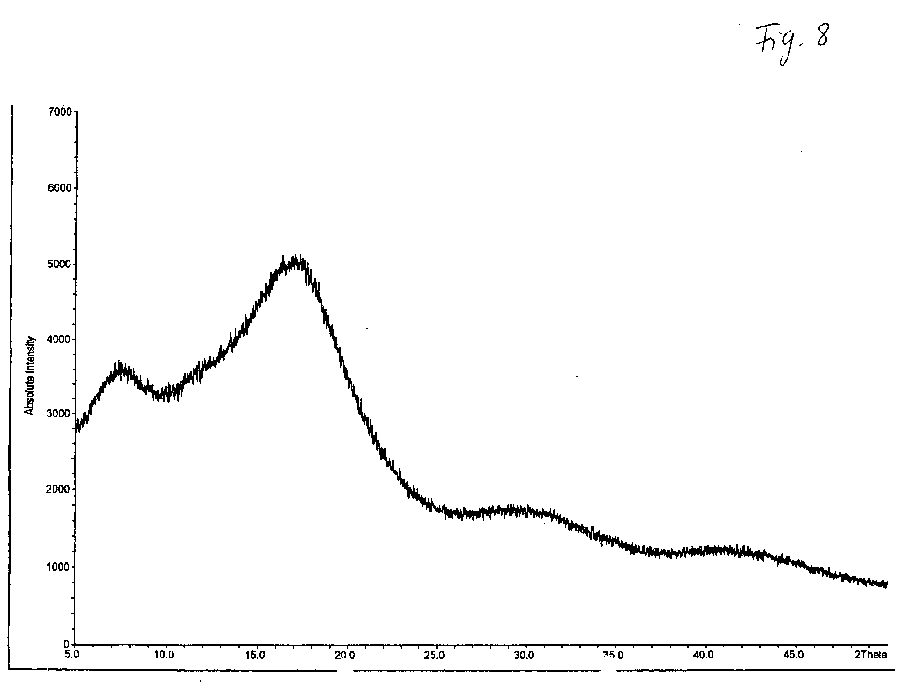

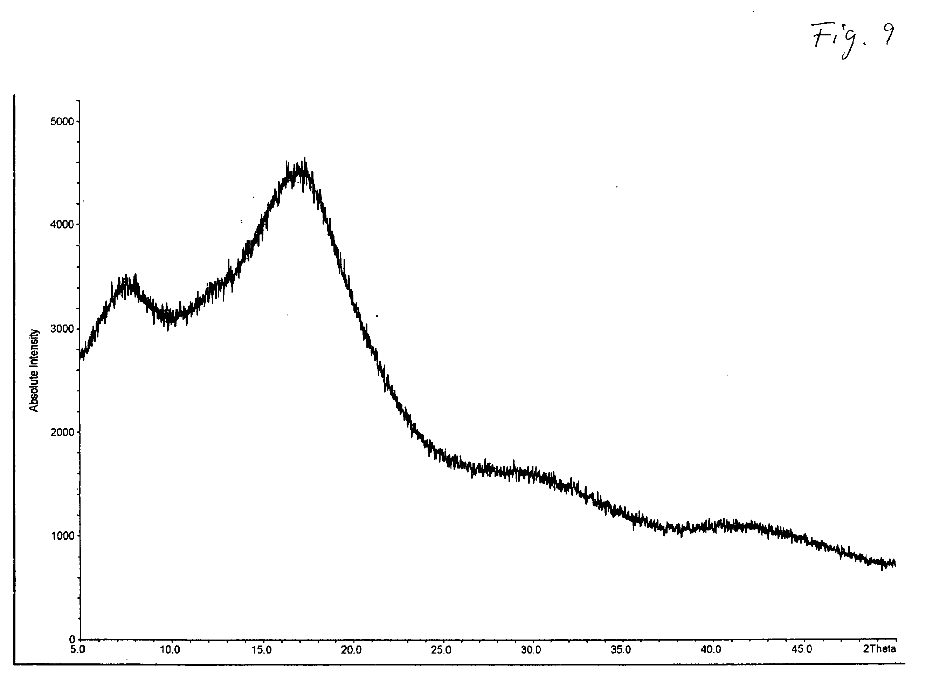

Zu einer Lösung von 1 g kristallinem Tibolon in 15 ml Aceton werden 4 g Polymethacrylat (Eudragit^{®} E) gelöst. Das Lösungsmittel wurde bei 30 bis 35° C, unter Anlegen eines Vakuums, verdampft. Der Gehalt der Probe an Tibolon wurde mittels HPLC bestimmt und lag bei 98,8 % des Soll-Gehaltes, die Summe aller Verunreinigungen (bezogen auf den Tibolon-Peak) betrug 0,16 %. Das Röntgenbeugungsdiagramm der Probe direkt nach Herstellung ist in Abbildung 8 dargestellt und weist nur amorphe Teile auf. Lagerung der Probe über 3 Monate bei 30° C führt zu keiner Veränderung der polymorphen Strukturen (siehe Abbildung 9); der Wirkstoffgehalt wurde zu 99,0 Gew.-% gemessen, und die Summe der Verunreinigungen betrug 0,29 Gew.-%.

## Patentansprüche

1. Verfahren zur Herstellung von Tibolon-haltigen Wirkstoffpulvern, enthaltend Tibolon in amorpher Form, **dadurch gekennzeichnet, dass** man von einer Lösung des Tibolons in wenigstens einem organischen Lösungsmittel, mit einem Gesamtwassergehalt des Lösungsmittels von nicht mehr als 15 %, bevorzugt von nicht mehr als 5 %, ausgeht, ein Trägermaterial, ausgewählt aus der Gruppe der Acrylpolymeren, ganz oder teilweise darin löst, und das Lösungsmittel entfernt.

2. Verfahren zur Herstellung von Tibolon-haltigen Wirkstoffpulvern nach Anspruch 1, enthaltend Tibolon in amorpher lagerstabiler Form, **dadurch gekennzeichnet, dass** man von einer Lösung des kristallinen Tibolons in wenigstens einem organischen Lösungsmittel, mit einem Gesamtwassergehalt des Lösungsmittels von nicht mehr als 15 %, bevorzugt von nicht mehr als 5 % ausgeht, ein Trägermaterial, ausgewählt aus der Gruppe der Acrylpolymeren, ganz oder teilweise darin löst, und das Lösungsmittel entfernt.

3. Verfahren nach Anspruch 1 oder 2, **dadurch gekennzeichnet, dass** das Trägermaterial aus der Gruppe pharmazeutischer Polymethacrylate, die aus Methacrylsäure-Copolymeren, Aminoalkylmethacrylat-Copolymeren, Methacrylsäurester-Copolymeren, Ammonioalkylmethacrylat-Copolymeren ausgewählt sind.

4. Verfahren gemäß irgendeinem der Ansprüche 1 bis 3, **dadurch gekennzeichnet, dass** man ein Verhältnis von Wirkstoff zu Träger im Bereich von 1:0,1 bis 10, insbesondere im Bereich von 1:0,5 bis 1:5 einstellt.

5. Verfahren gemäß einem oder mehreren der vorhergehenden Ansprüche 1 bis 4, **dadurch gekennzeichnet, dass** man als Lösungsmittel organische Lösungsmittel mit einem Gesamtwasseranteil von nicht mehr als 15 Vol.-%, alleine oder in Mischungen einsetzt, wobei die organischen Lösungsmittel ausgewählt sind aus der Gruppe der niederen Alkanole mit 1 bis 4 Kohlenstoffatomen, der Gruppe der Ether, der Ester und der Gruppe der alipathischen Ketone und deren Gemischen.

6. Verfahren gemäß Anspruch 5, **dadurch gekennzeichnet, dass** man als Lösungsmittel ein Lösungsmittel aus der Gruppe, bestehend aus Methanol, Ethanol, Isopropanol, n-Propanol, Aceton, Ethylacetat, Methylethylketon, Methyl-t-butylester, Dichlormethan, Petrolether, Hexan, einem Aceton/Wasser-Gemisch, einem Ethylacetat/Hexan-Gemisch, einem Gemisch aus Dichlormethan und Ethylacetat, einsetzt, wobei das Lösungsmittel insbesondere unter Inertgasatmosphäre gehalten wird und antioxidative und/oder basische Zusatzstoffe enthalten kann.

7. Verfahren gemäß einem der Ansprüche 1 bis 6, **dadurch gekennzeichnet, dass** man eine Wirkstofflösung einsetzt, die im Rahmen der Tibolonsynthese anfällt, und das Trägermaterial ganz oder teilweise darin gelöst wird.

8. Tibolon-haltige Wirkstoffpulver, enthaltend Tibolon in amorpher Form, **dadurch gekennzeichnet, dass** sie nach dem Verfahren gemäß einem oder mehreren der Ansprüche 1 bis 7 herstellbar sind.

9. Pharmazeutische Formulierung mit mindestens einem aktiven Wirkstoff und gegebenenfalls weiteren pharmazeutisch akzeptablen Hilfsstoffen, **dadurch gekennzeichnet, dass** sie als Wirkstoff Tibolon-haltige Wirkstoffpulver enthält, herstellbar gemäß einem oder mehreren der vorhergehenden Ansprüche 1 bis 7, wobei Tibolon ausschließlich in rein amorpher, lagerstabiler Form vorliegt.

10. Pharmazeutische Formulierung gemäß Anspruch 9, **dadurch gekennzeichnet, dass** sie in Form von Tabletten und Granulaten vorliegt, die auf bekannte Weise mit üblichen pharmazeutisch akzeptablen Hilfsstoffen herstellbar sind.

11. Pharmazeutische Formulierung gemäß Anspruch 9 oder 10 in Form von den Wirkstoff schnell freisetzenden, durch Direktverpressung hergestellten Tabletten.

12. Pharmazeutische Formulierung gemäß einem der Ansprüche 9 bis 11, **dadurch gekennzeichnet, dass** der Anteil an Bindemittel in der Gesamtmischung der Arzneimittelzubereitung bis zu 20 % (m/m) beträgt.

13. Pharmazeutische Zubereitung gemäß irgendeinem der Ansprüche 9 bis 11, **dadurch gekennzeichnet, dass** der Anteil an Füll- und Hilfsstoffen in der Gesamtmischung der Arzneimittelzubereitung 20 bis 99 Gew.-%, bevorzugt 50 bis 99 Gew.-%, beträgt.

14. Verwendung von rein amorphem Tibolon, herstellbar nach dem Verfahren gemäß irgendeinem der Ansprüche 1 bis 7, in morphologisch lagerstabiler Form zur Herstellung von oralen pharmazeutischen Formulierungen.

## Claims

1. Process for the preparation of powders of active components, containing tibolone in amorphous form, **characterized in that** starting from a solution of tibolone in at least one organic solvent having a total water content of said solvent of no more than 15 %, preferable of no more than 5 %, a carrier material selected from the group of acryl polymers is dissolved totally or partially therein and the solvent is removed.

2. Process for the preparation of powders of active components according to claim 1, containing tibolone in amorphous storage-stable form, **characterized in that** starting from a solution of crystalline tibolone in at least one organic solvent having a total water content of said solvent of no more than 15 %, preferably of no more than 5 %, a carrier material selected from the group of acryl polymers is dissolved totally or partially therein and the solvent is removed.

3. Process for the preparation of powders of active components according to claim 1 or 2, **characterized in that** said carrier material of the group of pharmaceutical polymethacrylates selected from methacrylic acid copolymers, aminoalkyl methacrylate copolymers, methacrylic acid ester copolymers, ammonioalkyl methacrylate copolymers.

4. Process for the preparation of powders of active components according to any of claims 1 to 3, **characterized in that** a ratio of agent to carrier is adjusted in the range of 1:0.1 to 10, preferably in the range of 1:0.5 to 1:5.

5. Process for the preparation of powders of active components according to one or more of preceding claims 1 to 4, **characterized in that** organic solvents having a total water content of no more than 15 vol%, solely or as mixture are used as the solvent, wherein the organic solvents are selected from the group of lower alkanols having 1 to 4 carbon atoms, the group of ethers, of esters, and the group of aliphatic ketones, and mixtures thereof.

6. Process for the preparation of powders of active components according to claim 5, **characterized in that** a solvent from the group consisting of methanol, ethanol, isopropanol, n-propanol, acetone, ethylacetate, methyl ethyl ketone, methyl t-butylester, dichloromethane, petroleum ether, hexane, an acetone water mixture, an ethylacetate hexane mixture, a mixture of dichloromethane and ethylacetate is used as the solvent, wherein the solvent is preferably stored in inert gas atmosphere and may contain antioxidative and/or basic additives.

7. Process for the preparation of powders of active components according to one of claims 1 to 6, **characterized in that** an agent solution accruing in the course of the synthesis of tibolone is used and the carrier material is totally or partially dissolved therein.

8. Powders of active components containing tibolone, containing tibolone in amorphous form, **characterized in that** they are preparable according to the method according to one or more of claims 1 to 7.

9. Pharmaceutical formulation having at least one active component and, if necessary, further pharmaceutical acceptable auxiliary substances, **characterized in that** it includes powders of active components containing tibolone as active component, preparable according to one or more of preceding claims 1 to 7, wherein tibolone is solely present in an exclusively amorphous, storage-stable form.

10. Pharmaceutical formulation according to claim 9, **characterized in that** it is in the form of tablets und granules, preparable with common pharmaceutical acceptable auxiliary substances in a known manner.

11. Pharmaceutical formulation according to claim 9 or 10, in the form of tablets fast releasing the agent and prepared by direct pressing.

12. Pharmaceutical formulation according to one of claims 9 to 11, **characterized in that** the proportion of binders in the total mixture of the drug preparation is up to 20 % (w/w).

13. Pharmaceutical preparation according to any of claims 9 to 11, **characterized in that** the proportion of fillers und auxiliary substances in the total mixture of the drug preparation is 20 to 99 wt%, preferably 50 to 99 wt%.

14. Use of exclusively amorphous tibolone preparable according to the process according to any of claims 1 to 7, in morphologically storage-stable form for preparing oral pharmaceutical formulations.

## Revendications

1. Procédé de préparation de poudres de principe actif contenant du Tibolon, qui contiennent le Tibolon sous forme amorphe, **caractérisé en ce que** l'on part d'une solution du Tibolon dans au moins un solvant organique, avec une teneur totale en eau du solvant de pas plus de 15 %, de préférence de pas plus de 5 %, que l'on dissout dedans totalement ou partiellement un matériau support choisi parmi le groupe des polymères acryliques, et que l'on élimine le solvant.

2. Procédé de préparation de poudres de principe actif contenant du Tibolon selon la revendication 1, contenant le Tibolon sous forme amorphe stable au stockage, **caractérisé en ce que** l'on part d'une solution du Tibolon cristallin dans au moins un solvant organique, avec une teneur totale en eau du solvant de pas plus de 15 %, de préférence de pas plus de 5 %, que l'on dissout dedans totalement ou partiellement un matériau support choisi parmi le groupe des polymères acryliques, et que l'on élimine le solvant.

3. Procédé selon la revendication 1 ou 2, **caractérisé en ce que** le matériau support est parmi le groupe des polyméthacrylates pharmaceutiques, qui sont choisis parmi les copolymères de l'acide méthacrylique, les copolymères de méthacrylates d'aminoalkyle, les copolymères d'esters d'acide méthacrylique, les copolymères de méthacrylates d'ammonio-alkyle.

4. Procédé selon l'une quelconque des revendications 1 à 3, **caractérisé en ce que** l'on règle un rapport du principe actif au support dans l'intervalle de 1 : 0,1 à 10, en particulier dans l'intervalle de 1 : 0,5 à 1 : 5.

5. Procédé selon l'une ou plusieurs des revendications précédentes 1 à 4, **caractérisé en ce que** l'on utilise comme solvants des solvants organiques avec une teneur totale en eau de pas plus de 15 % en volume, seuls ou en mélanges, sachant que les solvants organiques sont choisis parmi le groupe des alcanols « bas » avec 1 à 4 atomes de carbone, le groupe des éthers, des esters et le groupe des cétones aliphatiques et parmi leurs mélanges.

6. Procédé selon la revendication 5, **caractérisé en ce que** l'on utilise comme solvant un solvant du groupe constitué du méthanol, de l'éthanol, de l'isopropanol, du n-propanol, de l'acétone, de l'acétate d'éthyle, de la méthyléthylcétone, des esters de méthyl-t-butyle, du dichlorométhane, de l'éther de pétrole, de l'hexane, d'un mélange d'acétone et d'eau, d'un mélange d'acétate d'éthyle et d'hexane, d'un mélange de dichlorométhane et d'acétate d'éthyle, sachant que le solvant est maintenu en particulier sous une atmosphère de gaz inerte et peut contenir des additifs anti-oxydants et/ou basiques.

7. Procédé selon l'une des revendications 1 à 6, **caractérisé en ce que** l'on utilise une solution de principe actif qui est obtenue dans le cadre de la synthèse du Tibolon, et que l'on y dissout complètement ou partiellement le matériau support.

8. Poudre de principe actif contenant du Tibolon sous forme amorphe, **caractérisée en ce qu'**elle peut être préparée d'après le procédé selon l'une ou plusieurs des revendications 1 à 7.

9. Formulation pharmaceutique avec au moins un principe actif et, le cas échéant, d'autres substances auxiliaires pharmaceutiquement acceptables, **caractérisée en ce qu'**elle contient comme principe actif une poudre de principe actif contenant du Tibolon, pouvant être préparée selon l'une ou plusieurs des revendications précédentes 1 à 7, sachant que le Tibolon est présent exclusivement sous forme purement amorphe, stable au stockage.

10. Formulation pharmaceutique selon la revendication 9, **caractérisée en ce qu'**elle se présente sous forme de tablettes et de granulés qui peuvent être préparés d'une façon connue avec des substances auxiliaires habituelles pharmaceutiquement acceptables.

11. Formulation pharmaceutique selon la revendication 9 ou 10, sous forme de tablettes dégageant le principe actif rapidement, préparées par pressage direct.

12. Formulation pharmaceutique selon l'une des revendications 9 à 11, **caractérisée en ce que** la proportion de liant dans le mélange total de la préparation médicinale atteint jusqu'à 20 % (m/m).

13. Préparation pharmaceutique selon l'une quelconque des revendications 9 à 11, **caractérisée en ce que** la proportion de charges et de substances auxiliaires dans le mélange total de la préparation médicinale est de 20 à 99 % en poids, de préférence de 50 à 99 % en poids.

14. Utilisation de Tibolon purement amorphe, pouvant être préparé d'après le procédé selon l'une quelconque des revendications 1 à 7, dans une forme morphologiquement stable au stockage, pour la préparation de formulations pharmaceutiques orales.
